(19) Europäisches Patentamt / European Patent Office / Office européen des brevets

(11) **EP 2 280 078 B1**

(12) **EUROPEAN PATENT SPECIFICATION**

(45) Date of publication and mention of the grant of the patent:
**18.05.2016 Bulletin 2016/20**

(51) Int Cl.:
*C12Q 1/68* (2006.01)     *C12N 15/09* (2006.01)

(21) Application number: **09723964.4**

(22) Date of filing: **27.03.2009**

(86) International application number:
**PCT/JP2009/056293**

(87) International publication number:
**WO 2009/119809 (01.10.2009 Gazette 2009/40)**

(54) **MARKER FOR DETERMINATION OF BREAST CANCER, TEST METHOD, AND TEST KIT**

MARKER ZUR BESTIMMUNG VON BRUSTKREBS, TESTVERFAHREN UND TESTKIT

MARQUEUR POUR LA DÉTERMINATION DU CANCER DU SEIN, MÉTHODE D'ESSAI, ET KIT D'ESSAI

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **27.03.2008 JP 2008083897**

(43) Date of publication of application:
**02.02.2011 Bulletin 2011/05**

(73) Proprietors:
- **Kuroda, Masahiko**
  Tokyo 167-0052 (JP)
- **Miracure Inc.**
  Tokyo 191-0002 (JP)
- **Konica Minolta Holdings, Inc.**
  Tokyo 100-0005 (JP)

(72) Inventors:
- **TANAKA, Masami**
  Fukuoka-shi
  Fukuoka 814-0032 (JP)
- **OIKAWA, Kosuke**
  Kunitachi-shi
  Tokyo 186-0002 (JP)
- **MIZUTANI, Takayuki**
  Kuwana-shi
  Mie 511-0805 (JP)
- **TAKANASHI, Masakatsu**
  Isehara-shi
  Kanagawa 259-1114 (JP)

(74) Representative: **Raynor, Stuart Andrew**
J A Kemp
**14 South Square**
**Gray's Inn**
**London WC1R 5JJ (GB)**

(56) References cited:
WO-A1-2006/128245    WO-A2-2006/133022
WO-A2-2007/002375    WO-A2-2007/147409
WO-A2-2007/148235    US-A1- 2007 161 004

- LAWRIE CHARLES H ET AL: "Detection of elevated levels of tumour-associated microRNAs in serum of patients with diffuse large B-cell lymphoma", BRITISH JOURNAL OF HAEMATOLOGY, vol. 141, no. 5, 3 March 2008 (2008-03-03), pages 672-675, XP002545705, BLACKWELL PUBLISHING LTD ISSN: 1365-2141, DOI: 10.1111/J.1365-2141.2008.07077.X [retrieved on 2008-03-03]
- TRICOLI JAMES V ET AL: "MicroRNA: Potential for Cancer Detection, Diagnosis, and Prognosis.", CANCER RESEARCH, vol. 67, no. 10, 15 May 2007 (2007-05-15), pages 4553-4555, XP002642031, ISSN: 0008-5472
- BANDRÉS E ET AL: "Identification by Real-time PCR of 13 mature microRNAs differentially expressed in colorectal cancer and non-tumoral tissues", MOLECULAR CANCER, vol. 5, 1 January 2006 (2006-01-01), pages 29-38, XP002539258, BIOMED CENTRAL, LONDON, GB ISSN: 1476-4598, DOI: 10.1186/1476-4598-5-29 [retrieved on 2006-07-19]

- TANAKA MASAMI ET AL: "Down-regulation of miR-92 in human plasma is a novel marker for acute leukemia patients.", PLOS ONE, vol. 4, no. 5, E5532, May 2009 (2009-05), pages 1-5, XP002642032, ISSN: 1932-6203
- MITCHELL, P. S. ET AL.: 'Circulating microRNAs as stable blood-based markers for cancer detection.' PROCEEDINGS OF THE NATIONAL ACADEMY OF SCIENCES OF USA vol. 105, no. 30, 29 July 2008, pages 10513 - 10518, XP002545745
- NEGRINI, M. ET AL.: 'MicroRNAs in human cancer: from research to therapy.' JOURNAL OF CELL SCIENCE vol. 120, no. 11, 2007, pages 1833 - 1840, XP008123773
- IORIO, M. V. ET AL.: 'MicroRNA Gene Expression Deregulation in Human Breast Cancer' CANCER RESEARCH vol. 65, no. 16, 2005, pages 7065 - 7070, XP002603432
- LITMAN, T. ET AL.: 'MicroRNA profiling of breast cancer tissue using an LNA-based microarray.' NATURE METHODS vol. 4, no. 7, 2007, pages I - II, XP008143555
- LAWRIE, C. H. ET AL.: 'Detection of elevated levels of tumour-associated microRNAs in serum of patients with diffuse large B-cell lymphoma.' BRITISH JOURNAL OF HAEMATOLOGY vol. 141, no. 5, 03 March 2008, pages 672 - 675, XP002545705
- BREMNES, R. M. ET AL.: 'Circulating tumour-derived DNA and RNA markers in blood: a tool for early detection, diagnostics, and follow-up?' LUNG CANCER vol. 49, 2005, pages 1 - 12, XP025286820
- CALIN, G. A. ET AL.: 'MicroRNA signatures in human cancers' NATURE REVIEWS CANCER vol. 6, 2006, pages 857 - 866, XP002473506
- HUANG YONG ET AL: 'A bootstrap based analysis pipeline for efficient classification of phylogenetically related animal miRNAs' BMC GENOMICS, BIOMED CENTRAL LTD, LONDON, UK vol. 8, no. 1, 66, 06 March 2007, pages 1 - 9, XP021022373 DOI: 10.1186/1471-2164-8-66 ISSN: 1471-2164
- IORIO MARILENA V ET AL: "MicroRNA gene expression deregulation in human breast cancer", CANCER RESEARCH, AMERICAN ASSOCIATION FOR CANCER RESEARCH, US, vol. 65, no. 16, 15 August 2005 (2005-08-15), pages 7065-7070, XP002603432, ISSN: 0008-5472, DOI: 10.1158/0008-5472.CAN-05-1783
- ANONYMOUS: 'MicroRNA sequences', [Online] 01 January 2005, pages 1 - 4, XP055169881 Retrieved from the Internet: <URL:http://nar.oxfordjournals.org/content/suppl/2005/11/18/33.20.e179.DC1/gni178_S2.pdf> [retrieved on 2015-02-16]

## Description

## Technical Field

The present invention relates to a marker for diagnosis of breast cancer, a method of testing for breast cancer, and a test kit for breast cancer. More specifically, the present invention relates to a marker for detecting the onset of breast cancer or an early stage (clinical stage 0) of breast cancer, the marker being composed of a micro-RNA that is found in serum or plasma, a method of testing for breast cancer, and a test kit for breast cancer.

## Background Art

Breast cancer is a malignant tumor that is the most frequently found in females in many civilized countries, and the risk of such a malignant tumor progressing to invasive breast cancer still exceeds 10% for females of all ages (Non-Patent Document 1). This is partly attributed to the fact that cancer tissue cannot be detected by palpation or mammography examination until the cancer tissue grows to a certain size.

Hitherto, it has been believed that the onset and progression of cancers including breast cancer are caused by accumulation of multi-step mutations of oncogenes and tumor suppressor genes. Recently, it was found that so-called epigenetic mutations, such as DNA methylation and histone diacetylation, which do not involve mutations of the primary structure of genes also relate to the onset and progression of cancers.

It is becoming clear that abnormality of DNA methylation or histone modification is controlled by low-molecular-weight RNAs called micro-RNAs. It should be noted that a micro-RNA (hereinafter also referred to as "miRNA") is a small noncoding RNA that is composed of 19 to 25 nucleotides and that is not translated as an amino acid sequence of proteins. It has been confirmed that a large number of miRNAs are present in living organisms including humans, and they are systematically and highly conserved (Non-Patent Documents 2 to 4). In particular, in humans, about 800 types of miRNAs have been discovered to date (Non-Patent Documents 5 to 10).

Regarding the relationship between a cancer and a miRNA, Calin et al. reported that frequent down-regulation of miR-15 and miR-16, which are miRNAs, occurs in lymphocytic leukemia (Non-Patent Document 11). Furthermore, recently, Michael et al. reported that the expression of miR-143 and miR-145, which are miRNAs, decreases in human colorectal cancer (Non-Patent Document 12).

However, these reports do not describe whether or not the decrease in the expression of the miRNAs affects clinical pathological characteristics of cancers, and there are many unknown points regarding the expression regulation mechanism and expression abnormality of micro-RNAs in cancers, and target genes of the micro-RNAs.

[Non-Patent Document 1] Krupnick JG, Benovic JL: The role of receptor kinases and arrestins in G protein-coupled receptor regulation, Annu Rev Pharmacol Toxicol 1998, Vol. 38, p. 289-319

[Non-Patent Document 2] Ambros V, MicroRNA pathways in flies and worms: growth, death, fat, stress, and timing, Cell, 2003, Vol. 113, p. 673-6

[Non-Patent Document 3] Grosshans H, Slack FJ, Micro-RNAs: small is plentiful, J. Cell Biol., 2002, Vol. 156, p. 17-21

[Non-Patent Document 4] Nelson P, Kiriakidou M, Sharma A, Maniataki E, Mourelatos Z, The microRNA world: small is mighty, Trends Biochem. Sci., 2003, Vol. 28, p. 534-40

[Non-Patent Document 5] Lagos-Quintana M, Rauhut R, Lendeckel W, Tuschl T, Identification of novel genes coding for small expressed RNAs, Science, 2001, Vol. 294, p. 853-8

[Non-Patent Document 6] Lau NC, Lim LP, Weinstein EG, Bartel DP, An abundant class of tiny RNAs with probable regulatory roles in Caenorhabditis elegans, Science, 2001, Vol. 294, p. 858-62

[Non-Patent Document 7] Lee RC, Ambros V, An extensive class of small RNAs in Caenorhabditis elegans, Science, 2001, Vol. 294, p. 862-4

[Non-Patent Document 8] Mourelatos Z, Dostie J, Paushkin S et al., miRNP: miRNPs: a novel class of ribonucleoproteins containing numerous microRNAs, Genes Dev., 2002, Vol. 16, p. 720-8

[Non-Patent Document 9] Lim LP, Glasner ME, Yekta S, Burge CB, Bartel DP, Vertebrate microRNA genes, Science, 2003, Vol. 299, p. 1540

[Non-Patent Document 10] "Genome Summary List of Genome Currently Available", [online], miRBase, [Searched on March 27, 2009], Internet <URL: http://microrna.sanger.ac.uk/cgi-bin/targets/v5/genome.pl?order#by=genome#name>

[Non-Patent Document 11] Calin GA, Dumitru CD, Shimizu M et al., Frequent deletions and down-regulation of micro-RNA genes miR15 and miR16 at 13ql4 in chronic lymphocytic leukemia, Proc Natl. Acad. Sci. U.S.A., 2002, Vol. 99, p. 15524-9

[Non-Patent Document 12] Michael MZ, SM OC, van Holst Pellekaan NG, Young GP, James RJ, Reduced accumulation of specific microRNAs in colorectal neoplasia Mol Cancer Res, 2003, Vol. 1, p. 882-91

Lawie Charles et al. (2008) discloses the detection of elevated levels of tumor-associated micro-RNAs in serum of patients with diffuse large β-cell lymphoma.

**Disclosure of Invention**

**Problems to be Solved by the Invention**

An object of the present invention is to provide a marker which can detect the onset of breast cancer that cannot be detected by palpation or mammography examination and that is highly likely to be overlooked by existing pathological cell diagnosis or the presence of breast cancer cells even in an early stage (clinical stage 0) of breast cancer, and which is simple and has high reliability, the marker being composed of a micro-RNA that is present in serum or plasma; a test method; and a test kit.

**Means for Solving the Problems**

The inventors of the present invention have conducted intensive studies in order to solve the above problem or a problem that existing diagnostic markers for detecting breast cancer have low accuracy in terms of diagnostic sensitivity (sensitivity) and specificity and thus diagnosis cannot be accurately performed. As a result, it was found that the level of a micro-RNA (α) that is present in serum or plasma of a subject is significantly reduced after the onset of breast cancer, the level of a micro-RNA (β) that is present in serum or plasma is constant regardless of the affection of breast cancer, and the micro-RNAs can be used as a marker with which the onset of breast cancer or an early stage (clinical stage 0) of breast cancer can be diagnosed on the basis of the dynamics of these micro-RNAs. This finding led to the completion of the present invention. which is defined in the appended claims.

A marker of the present invention is a marker associated with breast cancer, and more specifically, (A) "a marker for detecting the onset of breast cancer or the early stage (clinical stage 0) of breast cancer" or a marker used as a prognostic factor of breast cancer, and is characterized by being (B) "a micro-RNA that is found in serum or plasma". In other words, the present invention provides a method in which the (B) is used as the (A).

The micro-RNA is preferably a micro-RNA (α) that is present in the serum or the plasma at a significantly reduced level after the onset of breast cancer, or during or after an early stage (during or after clinical stage 0) of breast cancer compared with that before the onset of breast cancer or before the early stage (before clinical stage 0) of breast cancer. The micro-RNA is at least one micro-RNA (α) selected from the group consisting of hsa-miR-92a1, hsa-miR-92a2, hsa-miR-22, hsa-miR-370, hsa-miR-601, hsa-miR-658, and hsa-miR-494.

In addition, the micro-RNA is preferably a combination of the micro-RNA (α) and a micro-RNA (β) that is present in the serum or the plasma at a constant level before and after the onset of breast cancer, or before, during, and after the early stage (before, during, and after clinical stage 0) of breast cancer. More preferably, the micro-RNA is a combination of the micro-RNA (α) and the micro-RNA (β), and a numerical value obtained by dividing the level of the micro-RNA (α) present in the serum or the plasma by the level of the micro-RNA (β) present in the serum or the plasma after the onset of breast cancer, or during or after the early stage (during or after clinical stage 0) of breast cancer is statistically significantly reduced compared with that before the onset of breast cancer or before the early stage (before clinical stage 0) of breast cancer.

The micro-RNA (β) is preferably at least one micro-RNA selected from the group consisting of hsa-miR-638, hsa-miR-630, and hsa-miR-572.

A method of testing for breast cancer as disclosed herein is characterized by including detecting the dynamics of a level of a micro-RNA that is present in serum or plasma derived from a subject and preferably includes (i) a step of comparing a numerical value obtained from a sample derived from a subject with a numerical value obtained from a sample derived from one or more healthy subjects or one or more breast cancer patients, each of the numerical values being obtained as a level of the micro-RNA (α) present in the serum or the plasma, to test whether or not there is a statistically significant difference between the numerical values, or (ii) a step of comparing a numerical value obtained from a sample derived from a subject with a numerical value obtained from a sample derived from one or more healthy subjects or one or more breast cancer patients, each of the numerical values being obtained as a normalized level of the micro-RNA (α) present in the serum or the plasma and determined by dividing a level of the micro-RNA (α) present in the serum or the plasma by a level of the micro-RNA (β) present in the serum or the plasma, to test whether or not there is a statistically significant difference between the numerical values. In particular, in steps (i) and (ii), the sample used as a comparison reference is more preferably a sample derived from one or more breast cancer patients.

Furthermore, a test kit for breast cancer is characterized by including at least a reagent for measuring the micro-RNA functioning as the marker of the present invention.

**Effect of the Invention**

According to the present invention, even the onset of breast cancer that cannot be detected by palpation or mammography examination and that is highly likely to be overlooked by existing pathological cell diagnosis or breast cancer in an early stage (clinical stage 0) can be easily tested by collecting the blood from a subject, and the difference between positivity and negativity is more significantly sensitive to that of existing breast cancer markers. Thus, the present disclosure can provide a marker, a test method, and a test kit which have high reliability. Furthermore, in the test of breast cancer, whether a subject is positive or negative can be determined by simply collecting the blood. This is preferable because the burden on the subject is low.

**Brief Description of Drawings**

[Fig. 1] Fig. 1 is a graph of the results shown in Table 5 of Example 3.
[Fig. 2] Fig. 2 is a graph showing the storage stability of hsa-miR-*92a evaluated by RT-PCR.
[Fig. 3] Fig. 3 is a graph showing the storage stability of hsa-miR-638 evaluated by RT-PCR.

**Best Modes for Carrying Out the Invention**

Next, a marker, a method of testing for breast cancer, and the use of a test kit for breast cancer of the present invention will be described in detail.

<Marker>

A marker of the present invention is a marker associated with breast cancer and is characterized by being a micro-RNA that is found in serum or plasma.

As described below, the marker of the present invention may be a micro-RNA ($\alpha$) alone or a combination of the micro-RNA ($\alpha$) and a micro-RNA ($\beta$).

[**Micro-RNA**]

Micro-RNAs (miRNAs) are small RNA molecules that do not code proteins, and it is believed that micro-RNAs relate to various biological phenomena such as development, differentiation, and proliferation. At least several hundred miRNA genes that code these small RNAs are present on the genome. First, an early miRNA having a length of several hundred to several thousand nucleotides is transcribed from the genes, and is then digested by a protein complex called a Microprocessor to produce a hairpin-shaped precursor miRNA composed of about 60 to 70 nucleotides. Subsequently, the precursor miRNA is moved from the nucleus to cytoplasm through exportin-5, and further digested by ribonuclease III called a Dicer to produce a mature miRNA dimer composed of 19 to 25 nucleotides. It is believed that the mature miRNA dimer forms a complex with Argonaute protein, which is associated with RNA interference (RNAi), and functions. The mechanism of the action thereof is believed to be partial (incomplete) hybridization with a messenger RNA, a part of which is (which is partially) complementary to a miRNA, and an unknown translation suppressing action concurrent with the hybridization. Accordingly, it is believed that the complex engages in a very unique expression control mechanism that one type of miRNA controls the translation of a plurality of messenger RNAs coding proteins.

Recently, the inventors detected a mature miRNA dimer, which should be present in cytoplasm, in serum or plasma. Although the mechanism through which miRNAs enter the bloodstream and the reason therefor are not completely clear, it was found that a level of a specific miRNA (micro-RNA ($\alpha$)) fluctuates whereas a level of a specific miRNA (micro-RNA ($\beta$)) is steady (constant).

In the present invention, for example, any of the numerical value (Copies/$\mu$L) measured by a real-time RT-PCR (or RT-qPCR) detection method and the signal intensity detected by a microarray analysis method or a northern blot analysis method can be used as an index as the "level".

(**Micro-RNA** ($\beta$))

Among mature miRNA dimers that are present in serum or plasma, the micro-RNA ($\beta$) is preferably at least one micro-RNA selected from the group consisting of hsa-miR-638, hsa-miR-630, and hsa-miR-572. The levels of these micro-RNAs are steady (constant) before and after the onset of breast cancer or before, during, and after an early stage (before, during, and after clinical stage 0) of breast cancer.

Accordingly, when the dynamics of the fluctuating level of the micro-RNA (α), which is caused by the affection of breast cancer or the progression of the stage of breast cancer, is detected, different samples can be normalized on the basis of the level of the micro-RNA (β) that is present in serum or plasma. That is, a numerical value obtained by dividing the level of the micro-RNA (α) that is present in serum or plasma by the level of the micro-RNA (β) that is present in serum or plasma is significantly reduced after the onset of breast cancer, or during or after the early stage (during or after clinical stage 0) of breast cancer as compared with the numerical value before the onset of breast cancer or before the early stage (clinical stage 0) of breast cancer. Therefore, the combination of the micro-RNAs (α) and (β) is suitable for the marker of the present invention.

The sequences of such micro-RNAs (β) are shown below.

(hsa-miR-638) agggaucgcgggcggguggcggccu
(hsa-miR-630) aguauucuguaccagggaaggu
(hsa-miR-572) guccgcucggcgguggcccca

**(Micro-RNA (α))**

Examples of the micro-RNA (α) include micro-RNAs such as hsa-miR-92a1, hsa-miR-92a2, hsa-miR-22, hsa-miR-370, hsa-miR-601, hsa-miR-658, and hsa-miR-494. The levels of these micro-RNAs are specifically reduced with canceration of cells derived from tissue of the breast, mammary ducts, and mammary glands (that is, the levels of these micro-RNAs that are present in serum or plasma are statistically significantly reduced after the onset of breast cancer, or during or after the early stage (clinical stage 0) of breast cancer compared with the levels before the onset of breast cancer or before the early stage (clinical stage 0) of breast cancer). Therefore, these micro-RNAs are suitable for a marker for detecting the onset of breast cancer or the early stage (clinical stage 0) of breast cancer.

These micro-RNAs (α) may be used alone or in combination of two or more types of micro-RNAs (α).

It should be noted that although hsa-miR-92a1 and hsa-miR-92a2 are transcribed from different genome regions, the sequences of the mature miRNAs are identical. Hereinafter, hsa-miR-92a1 and hsa-miR-92a2 are also referred to as "hsa-miR-*92a" in combination.

The sequences of such micro-RNAs (α) are shown below.

(hsa-miR-*92a) uauugcacuugucccggccugu
(hsa-miR-22) aagcugccaguugaagaacugu
(hsa-miR-370) gccugcugggguggaaccuggu
(hsa-miR-601) uggucuaggauuguuggaggag
(hsa-miR-658) ggcggagggaaguagguccguuggu
(hsa-miR-494) ugaaacauacacgggaaaccuc

[Breast cancer]

In this description, the "breast cancer" includes both ductal carcinoma originating from a terminal duct and lobular carcinoma originating from a lobule. It should be noted that the term "cancer" refers to a malignant tumor, but may be simply referred to as "tumor". The terms "malignant transformation" and "canceration" may be used as synonyms.

In this description, the term "onset" refers to the time when a subject is diagnosed as having a specified disease by comprehensive diagnosis on the basis of clinical symptoms specific to the disease, test data, and the like.

In this description, the term "prognostic factor" refers to determination information for forecasting the course of the disease after an operation, forecasting the future, and selecting an appropriate therapeutic method. In addition to the marker of the present invention, in general, the following factors are known as prognostic factors. Examples thereof include the size of a lump, the degree of metastasis to lymph nodes, the presence or absence of distant metastasis, the presence or absence of a hormone receptor (estrogen receptor (ER) or progesterone receptor (PgR)), the menopause status, and the nuclear grade.

In general, the clinical stages (stages) of breast cancer are defined by the terms a non-invasive cancer (intraepithelial carcinoma), a locally invasive cancer, a regional invasive cancer, and a distant metastatic cancer (metastatic cancer) or on the basis of the size of a tumor, the presence or absence of invasion to lymph nodes, and distant metastasis of cancer cells, and specifically represented as stages 0 to IV (clinical stages 0 to IV).

Non-invasive cancer (intraepithelial carcinoma): Non-invasive cancer refers to a cancer that stays in a local area, and corresponds to the earliest-stage cancer. Although the size of the cancer may significantly increase in the breast, invasion to surrounding tissue or metastasis to other sites in the body has not been caused. This cancer is usually detected by mammography examination.

Locally invasive cancer: Locally invasive cancer refers to a cancer that stays in the breast, though the cancer invades surrounding tissue.

Regional invasive cancer: Regional invasive cancer refers to a cancer that has invaded also tissue disposed around the breast, such as the chest wall or lymph nodes.

Distant metastatic cancer (metastatic cancer): Distant metastatic cancer (metastatic cancer) refers to a cancer that has spread by metastasis from the breast to other sites in the body.

Stage 0: The tumor is confined to a mammary duct or a mammary gland, and there is no invasion to surrounding mammary gland tissue (non-invasive cancer (intraepithelial carcinoma)). This is also referred to as the early stage of breast cancer.

Stage I: The diameter of the tumor is less than 2 cm, and metastasis outside the breast is not observed.

Stage II: The diameter of the tumor is 2 to 5 cm, and metastasis is observed in lymph nodes under the arm (one side or both sides) at the same side as that of the tumor.

Stage III: The diameter of a tumor exceeds 5 cm. Metastasis to lymph nodes is observed, and adhesion to surrounding tissue or adhesion to lymph nodes is observed. Alternatively, regardless of the size of the tumor, metastasis to the skin, chest wall, and thoracic lymph nodes at the downstream of the breast is observed.

Stage IV: Regardless of the size of the tumor, metastasis to organs and tissue such as the lung and bones distant from the breast or metastasis to lymph nodes of sites distant from the breast is observed.

In addition, breast cancer is classified on the basis of the type of tissue in which cancer cells are generated for the first time, and the range of the spread of the focus. A cancer originating from a mammary duct is referred to as "ductal carcinoma", and about 90% of breast cancer cases are ductal carcinoma. A cancer originating from a mammary gland is referred to as lobular carcinoma. A cancer originating from adipose tissue or connective tissue is referred to as "sarcoma". However, such a sarcoma is rare among breast cancer cases.

Ductal carcinoma in situ is a cancer that is confined in a mammary duct. Although this cancer has not invaded tissue around the breast, this cancer may spread along the mammary duct and the size thereof may increase in the breast. This cancer accounts for 20% to 30% of breast cancer cases.

Lobular carcinoma in situ proliferates inside a mammary gland. This cancer often generated in a plurality of sites of breasts at both sides. Lobular carcinoma in situ accounts for 1% to 2% of breast cancer cases.

Infiltrating ductal carcinoma is a cancer that originates from a mammary duct and that has invaded beyond a duct wall into surrounding mammary gland tissue. This cancer may spread by metastasis to a site other than the breast and accounts for 65% to 80% of breast cancer cases.

Infiltrating lobular carcinoma is a cancer that originates from a mammary gland, that invades into surrounding mammary gland tissue, and that further spreads by metastasis to a site other than the breast. This cancer accounts for 10% to 15% of breast cancer cases.

In addition, inflammatory breast cancer, which accounts for about 1% of all breast cancer cases, and Paget's disease of nipple are known. Furthermore, medullary carcinoma, tubular carcinoma, mucinous carcinoma (colloid carcinoma), and the like are also known as invasive cancers whose occurrence frequency is low, the invasive cancers originating from a mammary duct.

Breast cancer according to the present application include all carcinomas (cancers) originating from tissue of the breast, mammary ducts, and mammary glands, and non-invasive cancers in clinical stage 0 (intraepithelial carcinomas), which cannot be detected by palpation or mammography examination.

<Method of testing for breast cancer>

A method of testing for breast cancer of the present invention is characterized by including detection of the dynamics of a level of a micro-RNA that is present in serum or plasma derived from a subject and preferably includes step (i) or (ii) below.

Step (i): A step of comparing a numerical value obtained from a sample derived from a subject with a numerical value obtained from a sample derived from one or more healthy subjects or one or more breast cancer patients, each of the numerical values being obtained as a level of the micro-RNA ($\alpha$) present in the serum or the plasma, to test whether or not there is a statistically significant difference between the numerical values.

Step (ii): A step of comparing a numerical value obtained from a sample derived from a subject with a numerical value obtained from a sample derived from one or more healthy subjects or one or more breast cancer patients, each of the numerical values being obtained as a normalized level of the micro-RNA ($\alpha$) present in the serum or the plasma and determined by dividing a level of the micro-RNA ($\alpha$) present in the serum or the plasma by a level of the micro-RNA ($\beta$) present in the serum or the plasma, to test whether or not there is a statistically significant difference between the numerical values.

In the method of testing for breast cancer of the present invention, specifically, first, the total RNA is extracted from a specimen. In this description, the term "specimen" refers to the blood taken from a breast cancer patient (which refers to a human or a mammal other than a human which has been diagnosed as having breast cancer by pathological cell diagnosis), a healthy subject (which refers to a human or a mammal other than a human which has no specific chronic diseases and has no problem with their daily life and actions, and more specifically, a human or a mammal other than a human which has been diagnosed as not having a cancer during a regular physical checkup (including cancer screening) conducted once a year or more), a subject (which refers to a human or a mammal other than a human for which the onset of breast cancer is suspected), or the like.

Specifically, the blood is collected from a subject such as a human, and the supernatant obtained from the blood is then centrifuged to remove blood cells. The resulting blood is used as serum or plasma. The total RNA is extracted from the obtained serum or plasma. Examples of a method for extracting the total RNA include a guanidine-cesium chloride ultracentrifugation method, and an acid guanidinium-phenol-chloroform (AGPC) method.

Next, a miRNA according to the present invention contained in the extracted total RNA is detected. The detection method is not particularly limited as long as the amount of expression of the miRNA according to the present invention can be measured by the method. Examples of the method include a microarray analysis method, a northern blot analysis method, and a real-time RT-PCR detection method.

An example of the microarray analysis method is the method described in Example 1.

In the northern blot analysis method, the total RNA is separated by electrophoresis in accordance with the chain length, and transferred to a nitrocellulose membrane or a nylon membrane. This membrane is incubated in a buffer with a probe having a sequence complementary to the miRNA according to the present invention and labeled with a radioactive substance (e.g., $^{32}$P etc.) or the like. As a result, the miRNA according to the present invention hybridized with the probe can be detected on the basis of the label attached to the probe. It should be noted that each of prehybridization, hybridization, and a washing step is conducted under a stringent condition. Herein, the term "stringent condition" is a temperature of 37°C in a hybridization buffer containing 0.25 M sodium phosphate (pH 7.2), 7% SDS, and 0.5% sodium pyrophosphate, for example, when DNA labeled with $^{32}$P is used as the probe. In the washing step, the stringent condition is a temperature of 37°C in a washing buffer containing 2 x SSC and 1% SDS, and room temperature in a washing buffer containing 0.1 x SSC. In other cases, other standard conditions of the detection method may be used. When detection is performed by a radioactively labeled probe, the miRNA according to the present invention hybridized with the probe can be quantitatively determined using autoradiography on the basis of the band density.

Furthermore, according to the real-time RT-PCR (or RT-qPCR) detection method, the miRNA according to the present invention can be quantitatively detected by PCR using random priming cDNA corresponding to the total RNA and a primer complementary to the miRNA according to the present invention, via a fluorescent dye, such as SYBR Green, which is specifically bonded to double-strand DNA. The real-time RT-PCR detection method can be conducted in accordance with a known method or an instruction manual from a manufacturer of a detection device or a reagent (for example, ABI Prism 7900 Sequence Detection System (Perkin-Elmer Applied Biosystems), SYBR Green PCR Master Mix (Perkin-Elmer Applied Biosystems), or the like).

After the detection of the miRNA according to the present invention contained in the extracted total RNA, either step (i) or step (ii) is preferably conducted.

(**Step (i)**)

Step (i) is a step of comparing a numerical value obtained from a sample derived from a subject with a numerical value obtained from a sample derived from one or more healthy subjects or one or more breast cancer patients, each of the numerical values being obtained as a level of the micro-RNA ($\alpha$) present in the serum or the plasma, to test whether or not there is a statistically significant difference between the numerical values.

That is, step (i) is a step of comparing the absolute value of the amount of expression of at least one micro-RNA ($\alpha$) selected from the group consisting of hsa-miR-*92a, hsa-miR-22, hsa-miR-370, hsa-miR-601, hsa-miR-658, and hsa-miR-494; preferably hsa-miR-494, hsa-miR-370, and hsa-miR-*92a; and particularly preferably a micro-RNA ($\alpha$) of hsa-miR-494

(i-1) with the absolute value of the amount of expression of the micro-RNA ($\alpha$) obtained from a sample derived from one or more healthy subjects to test whether or not the former absolute value is statistically significantly low (where the significance level is preferably 5% and more preferably 1%), or
(i-2) with the absolute value of the amount of expression of a micro-RNA ($\alpha$) obtained from a sample derived from one or more breast cancer patients to test whether or not the former absolute value is statistically significantly high.

More specifically, in (i-1), as a result of the comparison, when the absolute value is statistically significantly low, a subject who provided the sample is diagnosed as having breast cancer. Conversely, when there is no statistically significant difference, the result of the comparison becomes useful information indicating that the subject has been diagnosed as a healthy subject. In (i-2), as a result of the comparison, when the absolute value is statistically significantly high, a subject who provided the sample is diagnosed as a healthy subject. Conversely, when there is no statistically significant difference, the result of the comparison becomes useful information indicating that the subject has been diagnosed as having breast cancer.

When the subject is one individual, preferably, a specimen is obtained in advance before the onset of breast cancer or before the early stage (before clinical stage 0) of breast cancer, and the amount of expression of the micro-RNA ($\alpha$) obtained from a sample derived from the specimen is used as a comparison target.

(**Step (ii)**)

Step (ii) is a step of comparing a numerical value obtained from a sample derived from a subject with a numerical value obtained from a sample derived from one or more healthy subjects or one or more breast cancer patients, each of the numerical values being obtained as a normalized level of the micro-RNA ($\alpha$) present in the serum or the plasma and determined by dividing a level of the micro-RNA ($\alpha$) present in the serum or the plasma by a level of the micro-RNA ($\beta$) present in the serum or the plasma, to test whether or not there is a statistically significant difference between the numerical values.

That is, step (ii) is a step of normalizing the amount of expression of a micro-RNA ($\alpha$) by dividing the amount of expression of at least one micro-RNA ($\alpha$) selected from the group consisting of hsa-miR-*92a, hsa-miR-22, hsa-miR-370, hsa-miR-601, hsa-miR-658, and hsa-miR-494; preferably a micro-RNA of hsa-miR-370 or hsa-miR-*92a by the amount of expression of at least one micro-RNA ($\beta$) selected from the group consisting of hsa-miR-638, hsa-miR-630, and hsa-miR-572, preferably hsa-miR-638, and comparing the obtained relative value of the amount of expression of the micro-RNA ($\alpha$)

(ii-1) with a relative value of the amount of expression of the micro-RNA ($\alpha$) obtained from a sample derived from one or more healthy subjects to test whether or not the former relative value is statistically significantly low (where the significance level is preferably 5% and more preferably 1%), or
(ii-2) with a relative value of the amount of expression of the micro-RNA ($\alpha$) obtained from a sample derived from one or more breast cancer patients to test whether or not the former relative value is statistically significantly high.

More specifically, in (ii-1), as a result of the comparison, when the relative value is statistically significantly low, the subject who provided the sample is diagnosed as having breast cancer. Conversely, when there is no statistically significant difference, the result of the comparison becomes useful information indicating that the subject has been diagnosed as a healthy subject. In (ii-2), as a result of the comparison, when the relative value is statistically significantly high, the subject who provided the sample is diagnosed as a healthy subject. Conversely, when there is no statistically significant difference, the result of the comparison becomes useful information indicating that the subject has been diagnosed as having breast cancer.

As in step (i), when the subject is one individual, preferably, a specimen is obtained in advance before the onset of breast cancer or before the early stage (clinical stage 0) of breast cancer, and the amount of expression of the micro-RNA ($\alpha$) obtained from a sample derived from the specimen is used as a comparison target.

Step (ii) is more preferable than step (i) because since normalization has been performed, the result does not depend on the type of micro-RNA ($\alpha$) used, the subject (individual), or whether or not the blood is collected before the onset of breast cancer or before the early stage (clinical stage 0) of breast cancer.

One embodiment of a specific procedure of step (ii) and the subsequent diagnosis of breast cancer will be exemplified below.

Normalization is performed by dividing the amount of expression of each of hsa-miR-*92a, hsa-miR-22, hsa-miR-370, hsa-miR-601, hsa-miR-658, and hsa-miR-494 by the amount of expression of hsa-miR-638, hsa-miR-630, or hsa-miR-572 corrected in advance by the signal intensity obtained by a microarray analysis method with an internal standard. That is, normalization is performed by dividing the level of the micro-RNA ($\alpha$) present in serum or plasma by the level of the micro-RNA ($\beta$) present in serum or plasma.

When the normalized amount of expression of hsa-miR-*92a, hsa-miR-22, hsa-miR-370, hsa-miR-601, hsa-miR-658, or hsa-miR-494 derived from a subject is significantly reduced as compared with the normalized amount of expression of hsa-miR-*92a, hsa-miR-22, hsa-miR-370, hsa-miR-601, hsa-miR-658, or hsa-miR-494 derived from a healthy subject, the subject can be diagnosed as having breast cancer. For example, the normalized amount of expression of hsa-miR-*92a, hsa-miR-22, hsa-miR-370, hsa-miR-601, hsa-miR-658, or hsa-miR-494 derived from a breast cancer patient is statistically significantly reduced as compared with the corrected amount of expression of hsa-miR-*92a, hsa-miR-22, hsa-miR-370, hsa-miR-601, hsa-miR-658, or hsa-miR-494 derived from a healthy subject.

As for a statistical method of testing whether or not there is a significant difference in step (i) or (ii), an appropriate method may be selected in accordance with the number of samples and the like. Examples of the method include a Ttest (T-test), an F-test, and a chi-square test.

By such a test method, even for a subject who is in clinical stage 0 of breast cancer and before the onset of breast cancer, it can be determined that the subject has breast cancer with high reliability. Therefore, it is possible to provide important information that may help in deciding what treatment strategy to follow such as medication or an operation.

<Test kit for breast cancer>

A test kit for breast cancer of the present disclosure is characterized by including at least a reagent for measuring a micro-RNA functioning as a marker of the present invention, wherein the test kit includes various instruments, materials, reagents and/or primers for amplifying genes, reagents related to gene amplification, and the like which are necessary for conducting the method of testing for breast cancer of the present invention.

Examples of the reagents for measuring a micro-RNA include various enzymes, buffering solutions, washing liquids, and dissolution liquids. Specifically, the reagents include at least a dissolution liquid for diluting a specimen and further include a primer for PCR, the primer being used for detecting the micro-RNA. A set of necessary instruments such as a microtiter plate and equipment for DNA amplification with which a plurality of specimens can be treated at the same time may further be included as kit elements.

According to a high throughput embodiment of the method of testing for breast cancer of the present invention, a microreactor element, specifically, a chip instrument may be included in the above test kit. Such an embodiment of an improved configuration is preferably a system that adopts a configuration in which digitized data with respect to signals obtained from a chip is taken in, a file is formed through computer processing, and the file is stored in a predetermined directory on a computer. Furthermore, the system may further include a capacity with which numerical data is statistically processed to display a significant difference from a control (experimental control). The data processing is performed using suitable software that enables statistical analysis through necessary correction and normalization. A person skilled in the art can establish a system for such a data processing using existing technologies, methods, and procedures.

EXAMPLES

Next, the present invention will be described in more detail by way of Examples.

[**Example 1**]

The blood was collected from respective healthy subjects (humans who were diagnosed as not having breast cancer during regular physical checkup including cancer screening and conducted once a year or more) and breast cancer patients (humans who were diagnosed as having breast cancer by pathological cell diagnosis) shown in Table 1, and serum was then separated from the blood. The total RNA was extracted from the serum using "(trade name) Isogen-LS" (produced by Nippon Gene Co., Ltd.), and the concentration of the total RNA was adjusted to 100 ng/$\mu$L.

Next, dephosphorylation reaction of the total RNA was conducted using "(trade name) Alkaline Phosphatase (Calf intestine) (CIAP)" (produced by Takara Bio Inc.), which is an alkaline phosphatase derived from calf small intestine. Subsequently, labeling was performed with cyanine, which is a dye, using "(trade name) T4 RNA Ligase (Cloned)" (produced by Ambion, Inc.). The above operation was conducted using "(trade name) miRNA Labeling Reagent and Hybridization Kit (catalogue No.: 5190-0408)" (produced by Agilent technologies) in accordance with the protocol attached thereto.

Furthermore, hybridization of the total RNA labeled with cyanine was conducted using a microarray slide of "(trade name) Human miRNA Microarray kit 8 x 15K V2 (Catalogue No.: G4470B) (produced by Agilent technologies) to obtain signals.

Subsequently, the microarray slide was scanned using "(trade name) DNA Microarray Scanner" (produced by Agilent technologies). For signal detection, "Feature Extraction 9.5.3 Software" and "Agilent Scan Control Software (ver. 7.0)" that were attached to the above scanner were used.

The obtained results are shown in Table 1.

[Table 1]

Table 1: Example 1

| Sample No. | Derivation of specimen | | Micro-RNA(α)[hsa-miR-] | | | | | | Micro-RNA (β) [hsa-miR-] | | |
|---|---|---|---|---|---|---|---|---|---|---|---|
| | | | *92a | 22 | 370 | 601 | 658 | 494 | 638 | 630 | 572 |
| 1 | Healthy subject | Male | 86.0328 | - | - | - | - | 24.1678 | 891.496 | - | 40.5002 |
| 2 | | Female | 164.835 | - | - | - | - | 38.4196 | 2842.82 | - | 115.124 |
| 3 | | Male | 555.067 | - | - | - | - | 22.2111 | 7400.18 | - | 141.962 |
| 4 | | Female | 4.9112 | 1.1041 | 13.348 | 6.5309 | 5.1405 | - | 781.85 | 301.89 | - |
| 5 | | Female | 86.033 | 19.435 | 23.929 | 11.651 | 7.1342 | - | 891.5 | 368.61 | - |
| 6 | | Female | 164.84 | 54.97 | 53.94 | 24.107 | 17.603 | - | 2842.8 | 902.63 | - |
| 7 | | Female | 37.789 | 8.7482 | 6.4142 | 5.9387 | 3.0777 | - | 582.09 | 168.4 | - |
| 8 | | Female | 555.07 | 68.946 | 75.813 | 114.91 | 60.459 | - | 7400.2 | 4352.3 | - |
| 9 | | Female | 27.639 | 12.887 | 8.6969 | 6.1194 | 0.4488 | - | 3139.8 | 223.07 | - |
| 10 | | Female | 1.1296 | 4.4697 | 5.6448 | 3.7716 | 1.3054 | - | 1933.5 | 231.36 | - |
| 11 | Breast cancer patient | Female | 0.146762 | - | - | - | - | -1.95187 | 2598.58 | - | 161.39 |
| 12 | | Female | 3.64585 | - | - | - | - | -1.66446 | 4297.62 | - | 247.888 |
| 13 | | Female | 1.1017 | 0.001 | 6.0851 | 18.115 | 10.246 | - | 2145.8 | 414.7 | - |
| 14 | | Female | 7.7428 | 0.897 | 9.0196 | 28.427 | 18.597 | - | 3911.7 | 752.39 | - |
| 15 | | Female | 0.001 | 0.001 | 0.001 | 0.3747 | -0.6201 | - | 1044.48 | 49.9934 | - |
| 16 | | Female | 3.64585 | 0.33198 | 4.21704 | 3.29853 | -0.2897 | - | 4297.62 | 329.343 | - |
| 17 | | Female | 0.001 | 2.1077 | 1.3858 | 2.0594 | 1.5543 | - | 801.72 | 72.411 | - |
| 18 | | Female | 37.118 | 36.601 | 6.7462 | 6.2106 | 0.0422 | - | 3534.5 | 264.92 | - |
| 19 | | Female | 0.001 | 0.8274 | 0.5354 | 2.704 | 1.415 | - | 1028.7 | 49.713 | - |
| 20 | | Female | 0.001 | 0.001 | 0.001 | 0.001 | -14.34 | - | 10289 | 514.33 | - |
| 21 | | Female | 2.9398 | 0.1292 | 0.8485 | 1.7492 | 0.7915 | - | 1184.2 | 113.6 | - |

[EXAMPLE 2]

A T-test (significant difference test) of the averages of the healthy subjects and the breast cancer patients was conducted on the basis of the analysis data obtained in Example 1. Specifically, the T-test was conducted using the numerical values of hsa-miR-92a, hsa-miR-22, hsa-miR-370, hsa-miR-601, and hsa-miR-658 obtained from the subjects (sample Nos. 4 to 10) and the breast cancer patients (sample Nos. 13 to 21) as they are. The obtained p-values are shown in Table 2. Furthermore, the numerical values of hsa-miR-92a, hsa-miR-22, hsa-miR-370, hsa-miR-601, and hsa-miR-658 obtained from the healthy subjects (sample Nos. 4 to 10) and the breast cancer patients (sample Nos. 13 to 21) shown in Table 1 were divided by hsa-miR-638 obtained from the corresponding healthy subjects (sample Nos. 4 to 10) and breast cancer patients (sample Nos. 13 to 21), and the T-test was conducted. The obtained p-values are shown in Table 2.

[Table 2]

Table 2: Example 2

|  | Micro-RNA ($\alpha$) [hsa-miR-] | | | | |
|---|---|---|---|---|---|
|  | *92a | 22 | 370 | 601 | 658 |
| p-Value that is divided (normalized) by micro-RNA ($\beta$) = hsa-miR-638 | 0.026606507 | 0.030637942 | 0.012629431 | 0.028309554 | 0.027608012 |
| p-Value that is not divided by micro-RNA ($\beta$) | 0.161400855 | 0.105371999 | 0.063186344 | 0.295651814 | 0.215317149 |

Referring to Table 2, as a result of the significant difference test of the averages of the healthy subjects and the breast cancer patients, the p-values of all the markers normalized by hsa-miR-638 satisfied $p < 0.05$, and thus a significant difference was observed. Accordingly, Table 2 showed that breast cancer can be diagnosed more accurately by normalizing the markers of hsa-miR-370, hsa-miR-601, hsa-miR-92, hsa-miR-22, and hsa-miR-658 with hsa-miR-638.

[COMPARATIVE EXAMPLE 1]

Sample Nos. i to iv shown in Table 3 were each quantified by a predetermined immunoassay method using, as specimens, the bloods (serums or plasmas) derived from four breast cancer patients among sample Nos. 13 to 21 and, as markers, CA15-3, CEA, NCC-ST-439, and BCA225, all of which have been hitherto used. The obtained results are shown in Table 3.

[Table 3]

Table 3: Comparative Example 1

| No. Sample No. | | Existing breast cancer marker | | | |
|---|---|---|---|---|---|
|  |  | CA15-3 | CEA | NCC-ST-439 | BCA225 |
| i | | 31 | 1.9 | 7.2 | 95 |
| ii | | 9 | 1 | 2.1 | 55 |
| iii | | 14.1 | 1.3 | 1.7 | 130 |
| iv | | 3.9 | 2.8 | 2.4 | ≤ 30 |
| Remarks | Reference value | 25 U/mL or less | 5.0 ng/mL or less | 7.0 U/mL or less | 160 U/mL or less |
|  | Antigen (Protein) | Sugar chain antigen 15-3 | Carcinoembryonic antigen | Sialic acid sugar chain antigen | Mucin-type glycoprotein |
|  | Assay method | CLEIA | | EIA | |

Referring to Table 3, except for CA15-3 and NCC-ST-439 of sample No. i, all the obtained results satisfied the index of the reference value of the existing breast cancer markers. In other words, when breast cancer was diagnosed using the existing breast cancer markers, all samples were diagnosed as false negative, and consequently, breast cancer could not be determined.

[**EXAMPLE 3**]

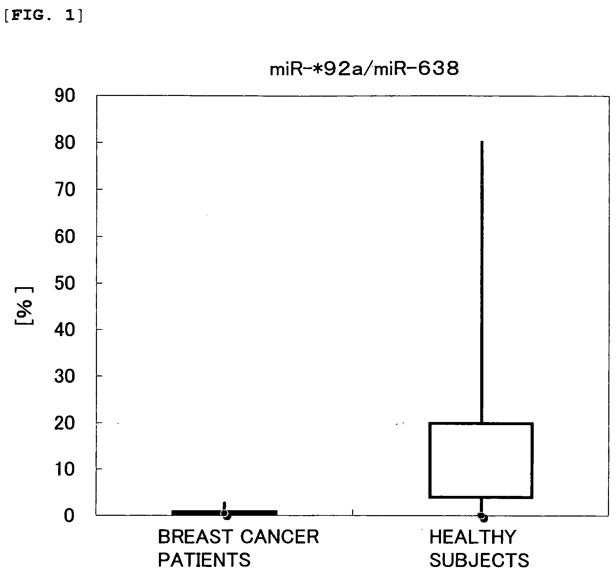

The blood was collected from respective healthy subjects and breast cancer patients, and the serum was separated from the blood. The total RNA was extracted from the serum using "(trade name) Isogen-LS" (produced by Nippon Gene Co., Ltd.). Subsequently, hsa-miR-*92a and hsa-miR-638 were quantified by RT-PCR using the total RNA extracted from each of the samples. The measurement of the RT-PCR was conducted using "(trade name) TaqMan MicroRNA Assay Kit (produced by Applied Biosystems Japan Ltd.)" in accordance with a protocol attached thereto. The results thereof are shown in Tables 4 to 7 and Fig. 1.
[**Table 4**]

Table 4: hsa-miR-*92a alone

|  | Median value | 0.25 | 0.75 | Maximum | Minimum | The number of samples |
|---|---|---|---|---|---|---|
| Breast cancer patients | 0.002056 | 0.000532 | 0.522054 | 0.166300 | 0.000005 | 17 |
| Healthy subjects | 0.019906 | 0.006089 | 0.038408 | 0.199123 | 0.000346 | 28 |

[**Table 5**]

Table 5: hsa-miR-92a/hsa-miR-638

|  | Median value | 0.25 | 0.75 | Maximum | Minimum | The number of samples |
|---|---|---|---|---|---|---|
| Breast cancer patients | 0.385240 | 0.189680 | 0.792999 | 2.696266 | 0.005926 | 17 |
| Healthy subjects | 10.263893 | 3.935504 | 19.925313 | 80.168510 | 0.649503 | 28 |

[**Table 6**]

Table 6: hsa-miR-*92a alone

|  |  | Breast cancer patients | Healthy subjects |
|---|---|---|---|
| Test | Positive | 12 | 7 |
|  | Negative | 5 | 21 |

Note that the diagnostic sensitivity (sensitivity) and the specificity are calculated as follows.

$$\text{Diagnostic sensitivity} = 12/(12 + 5) \times 100 = 70.6\%$$

$$\text{Specificity} = 21/(7 + 21) \times 100 = 75.0\%$$

(However, the threshold is assumed to be 0.25.)
[**Table 7**]

Table 7: hsa-miR-*92a/638 (normalized)

|  |  | Breast cancer patients | Healthy subjects |
|---|---|---|---|
| Test | Positive | 17 | 7 |
|  | Negative | 0 | 21 |

Note that the diagnostic sensitivity (sensitivity) and the specificity are calculated as follows.

$$\text{Diagnostic sensitivity} = 17/(17 + 0) \times 100 = 100.0\%$$

$$\text{Specificity} = 21/(7 + 21) \times 100 = 75.0\%$$

(However, the threshold is assumed to be 0.25.)

Table 6 shows the results of the test using the values of hsa-miR-*92a obtained by taking serum from respective 28 healthy subjects and 17 breast cancer patients. When 25% of the value obtained in healthy subject samples was set to the threshold, the diagnostic sensitivity was 70.6% and the specificity was 75%. Thus, hsa-miR-*92a is a marker that is excellent in terms of diagnostic sensitivity and specificity as compared with generally used breast cancer markers, which have a diagnostic sensitivity of about 20% to 40% and a specificity of about 30% to 50%.

Table 7 shows the results of the test using the values obtained by dividing hsa-miR-*92a by hsa-miR-638. When 25% of the value obtained in healthy subject samples was similarly set to the threshold, the diagnostic sensitivity was 100%. Thus, breast cancer could be diagnosed with high accuracy as compared with the case where the test was conducted using hsa-miR-*92a only.

[**REFERENCE EXAMPLE 1**]

The storage stability of hsa-miR-*92a and hsa-miR-638 obtained by separating serums from the bloods of healthy subjects was evaluated.

As for a specific procedure, first, the serums were left to stand at room temperature (25°C) for seven days. Next, a change in the amount of each of the micro-RNAs with time was quantitatively determined by RT-PCR as in Example 3.

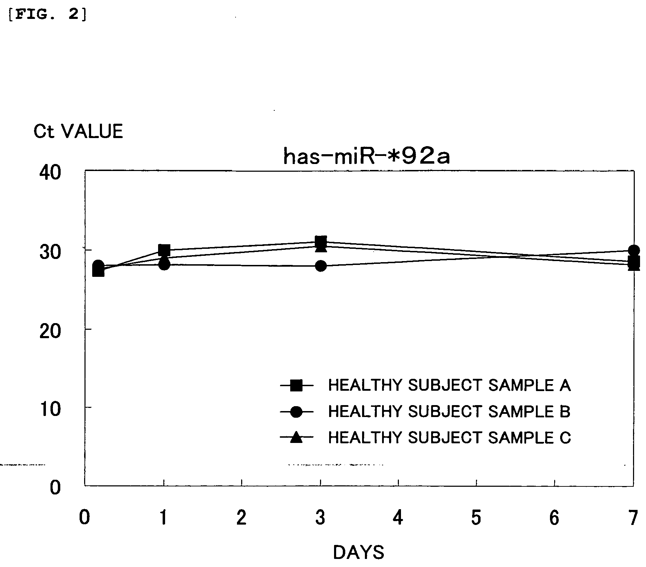

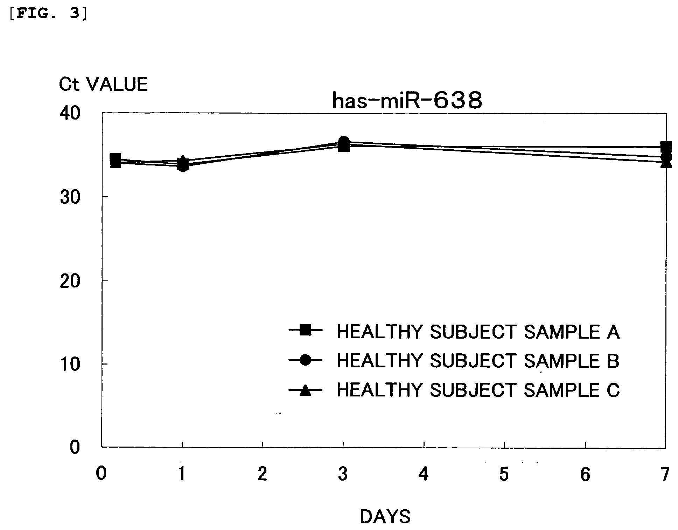

The results obtained with regard to hsa-miR-*92a are shown in Fig. 2, and the results obtained with regard to hsa-miR-638 are shown in Fig. 3.

Referring to Figs. 2 and 3, the Ct value of all samples did not substantially change for seven days, indicating that the micro-RNAs in serum are very stable. These results show that the micro-RNAs in serum can be satisfactorily used as a marker used for determining breast cancer in clinical diagnosis.

**Industrial Applicability**

The marker of the invention of the subject application can easily detect the onset of breast cancer that cannot be detected by palpation or mammography examination and that is highly likely to be overlooked by existing pathological cell diagnosis or breast cancer in an early stage (clinical stage 0) with high reliability. Therefore, the marker of the invention is suitable for use in a test kit for breast cancer, the test kit including at least a reagent for measuring a micro-RNA.

**Claims**

1. A method of testing for breast cancer comprising:

   (i) detecting the expression of a micro-RNA ($\alpha$) and a micro-RNA ($\beta$) in total RNA extracted from serum or plasma derived from a subject,
   wherein the micro-RNA ($\alpha$) is a micro-RNA that is present in the serum or the plasma at a significantly reduced level after the onset of breast cancer, or during or after an early stage (during or after clinical stage 0) of breast cancer compared with that before the onset of breast cancer or before the early stage (before clinical stage 0) of breast cancer,
   wherein the micro-RNA ($\beta$) is a micro-RNA that is present in the serum or the plasma at a constant level before and after the onset of breast cancer, or before, during, and after the early stage (before, during, and after clinical stage 0) of breast cancer; and
   (ii) obtaining a numerical value as a normalized level of the micro-RNA ($\alpha$) by dividing the level of the micro-RNA ($\alpha$) by the level of the micro-RNA ($\beta$) and using the normalized level of micro-RNA as a marker of breast cancer;

   wherein the micro-RNA ($\alpha$) is at least one type of micro-RNA selected from hsa-miR-92a1, hsa-miR92a2, hsa-miR-22, hsa-miR-370, hsa-miR-601, hsa-miR-658 and hsa-miR-494.

2. The method according to claim 1, further comprising:

(iii) comparing the numerical value obtained from step (ii) with a numerical value obtained from a sample derived from one or more healthy subjects or one or more breast cancer patients, each of the numerical values being obtained as a normalized level of the micro-RNA (α) and determined by dividing a level of the micro-RNA (α) by a level of the micro-RNA (β) to test whether or not there is a statistically significant difference between the numerical values,

thereby determining whether or not the subject has breast cancer.

3. The method according to claim 1 or 2, wherein the micro-RNA (α) is at least one type of micro-RNA selected from hsa-miR-92a1, hsa-miR-92a2, hsa-miR-22, hsa-miR-370, hsa-miR-601 and hsa-miR-658.

4. The method according to any one of claims 1 to 3, wherein the micro-RNA (β) is at least one type of micro-RNA selected from hsa-miR-638, hsa-miR-630, and hsa-miR-572.

5. The method according to any one of claims 1 to 4, wherein the numerical value obtained in step (ii) is compared with a numerical value obtained from a sample derived from one or more breast cancer patients.

6. The method according to any one of claims 2 to 5, wherein the numerical value obtained in step (ii) is compared with a numerical value obtained from a sample derived from one or more breast cancer patients and the subject is diagnosed as having breast cancer when it is determined in step (iii) that there is no statistically significant difference.

7. The method according to any one of Claims 1 to 6, wherein the step of detecting the expression of a micro-RNA (α) and/or a micro-RNA (β) is performed by a microarray analysis, northern blot and/or real time RT-PCR method.

8. The method according to any one of Claims 1 to 7, wherein total RNA is extracted by a guanidine-cesium chloride ultracentriguation or acid guanidinium-phenol-chloroform (AGPC) method.

9. Use of a kit in a method of testing for breast cancer according to any one of claims 1 to 8, wherein the kit comprises at least a reagent for measuring a micro-RNA which includes a PCR primer for detecting a micro-RNA (α) and a PCR primer for detecting a micro-RNA (β), wherein the micro-RNA (α) is selected from hsa-miR-92a1, hsa-miR-92a2, hsa-miR-22, hsa-miR-370, hsa-miR-601, hsa-miR-658, and hsa-miR-494.

10. The use according to claim 9, wherein the micro-RNA (β) is selected from hsa-miR-638, hsa-miR-630, and hsa-miR-572.

**Patentansprüche**

1. Verfahren zum Testen auf Brustkrebs umfassend:

(i) Detektieren der Expression einer MikroRNA (α) und einer MikroRNA (β) in der Gesamt-RNA, die von Serum oder Plasma, welches von einem Subjekt stammt, extrahiert wird,
wobei die MikroRNA (α) eine MikroRNA ist, die im Serum oder Plasma auf einem signifikant verringerten Niveau nach Einsetzen von Brustkrebs vorliegt, oder während oder nach einem frühen Stadium (während oder nach dem klinischen Stadium 0) von Brustkrebs, verglichen mit dem vor Einsetzen des Brustkrebses oder vor dem frühen Stadium (vor dem klinischen Stadium 0) von Brustkrebs,
wobei die MikroRNA (β) eine MikroRNA ist, die in dem Serum oder dem Plasma auf einem konstanten Niveau vor oder nach dem Einsetzen von Brustkrebs, oder vor, während und nach dem frühen Stadium (vor, während und nach dem klinischen Stadium 0) von Brustkrebs vorliegt; und
(ii) Erhalten eines numerischen Wertes als normalisiertes Niveau der MikroRNA (α) durch Teilen des Niveaus der MikroRNA (α) durch das Niveau der MikroRNA (β) und Verwendung des normalisierten Niveaus an MikroRNA als Marker für Brustkrebs;

wobei die MikroRNA (α) mindestens eine Art von MikroRNA ist, ausgewählt aus hsa-miR-92a1, hsa-miR92a2, hsa-miR-22, hsa-miR-370, hsa-miR-601, hsa-miR-658 und hsa-miR-494.

**2.** Verfahren gemäß Anspruch 1, ferner umfassend:

(iii) Vergleichen des numerischen Wertes, welcher in Schritt (ii) erhalten wurde, mit einem numerischen Wert, welcher aus einer Probe erhalten wurde, die von einem oder mehreren gesunden Subjekt(en) oder einem oder mehreren Brustkrebspatienten stammt, wobei jeder der numerischen Werte als normalisiertes Niveau der Mi-kroRNA ($\alpha$) erhalten wurde und durch Teilen eines Niveaus der MikroRNA ($\alpha$) durch ein Niveau der MikroRNA ($\beta$) bestimmt wurde, um zu testen ob es eine statistisch signifikante Differenz zwischen den numerischen Werten gibt oder nicht,

wodurch bestimmt wird, ob das Subjekt Brustkrebs hat oder nicht.

**3.** Verfahren gemäß Anspruch 1 oder 2, wobei die MikroRNA ($\alpha$) mindestens eine Art von MikroRNA ist, ausgewählt aus hsa-miR-92a1, hsa-miR-92a2, hsa-miR-22, hsa-miR-370, hsa-miR-601 und hsa-miR-658.

**4.** Verfahren gemäß einem der Ansprüche 1 bis 3, wobei die MikroRNA ($\beta$) mindestens eine Art von MikroRNA ist, ausgewählt aus hsa-miR-638, hsa-miR-630 und hsa-miR-572.

**5.** Verfahren gemäß einem der Ansprüche 1 bis 4, wobei der numerische Wert, welcher in Schritt (ii) erhalten wurde, mit einem numerischen Wert verglichen wird, welcher aus einer Probe erhalten wurde, die von einem oder mehreren Brustkrebspatienten stammt.

**6.** Verfahren gemäß einem der Ansprüche 2 bis 5, wobei der numerische Wert, welcher in Schritt (ii) erhalten wurde, mit einem numerischen Wert, welcher von einer Probe erhalten wurde, die von einem oder mehreren Brustkrebspatienten stammt, verglichen wird, und dem Subjekt das Vorliegen von Brustkrebs diagnostiziert wird, wenn in Schritt (iii) bestimmt wird, dass keine statistisch signifikante Differenz vorliegt.

**7.** Verfahren gemäß einem der Ansprüche 1 bis 6, wobei der Schritt des Detektierens der Expression einer MikroRNA ($\alpha$) und/oder einer MikroRNA ($\beta$) durch eine Mikroarray-Analyse, Northern-Blot und/oder Echtzeit RT-PCR-Verfahren durchgeführt wird.

**8.** Verfahren gemäß einem der Ansprüche 1 bis 7, wobei die Gesamt-RNA durch eine Guanidin-Cäsiumchlorid-Ultrazentrifugation oder saure Guanidinium-Phenol-Chloroform (AGPC)-Methode extrahiert wird.

**9.** Verwendung eines Kits bei einem Verfahren zum Testen auf Brustkrebs gemäß einem der Ansprüche 1 bis 8, wobei das Kit mindestens ein Reagens zur Messung einer MikroRNA umfasst, welches einen PCR-Primer zur Detektion einer MikroRNA ($\alpha$) und einen PCR-Primer zur Detektion einer MikroRNA ($\beta$) umfasst, wobei die MikroRNA ($\alpha$) ausgewählt ist aus hsa-miR-92a1, hsa-miR-92a2, hsa-miR-22, hsa-miR-370, hsa-miR-601, hsa-miR-658 und hsa-miR-494.

**10.** Verwendung gemäß Anspruch 9, wobei die MikroRNA ($\beta$) ausgewählt ist aus hsa-miR-638, hsa-miR-630 und hsa-miR-572.

**Revendications**

**1.** Procédé de test de cancer du sein, comportant les étapes suivantes :

i) détecter l'expression d'un micro-ARN ($\alpha$) et d'un micro-ARN ($\beta$), dans l'ARN total extrait d'un sérum ou plasma prélevé chez un suj et,
étant entendu que le micro-ARN ($\alpha$) est un micro-ARN qui est présent dans le sérum ou le plasma à un niveau significativement réduit après le début d'un cancer du sein ou pendant ou après un stade précoce (pendant ou après le stade clinique 0) d'un cancer du sein, comparé à celui d'avant le début d'un cancer du sein ou d'avant un stade précoce (d'avant le stade clinique 0) d'un cancer du sein,
et étant entendu que le micro-ARN ($\beta$) est un micro-ARN qui est présent dans le sérum ou le plasma à un niveau constant avant et après le début d'un cancer du sein, ou avant, pendant ou après un stade précoce (avant, pendant ou après le stade clinique 0) d'un cancer du sein ;
ii) et calculer une valeur numérique, en tant que niveau normalisé du micro-ARN ($\alpha$), en divisant le niveau du micro-ARN ($\alpha$) par le niveau du micro-ARN ($\beta$), et se servir de ce niveau normalisé de micro-ARN comme d'un marqueur de cancer du sein ;

et dans lequel procédé le micro-ARN (α) est un micro-ARN d'au moins un type choisi parmi les suivants : hsa-miR-92a1, hsa-miR-92a2, hsa-miR-22, hsa-miR-370, hsa-miR-601, hsa-miR-658 et hsa-miR-494.

2. Procédé conforme à la revendication 1, qui comporte en outre l'étape suivante :

iii) comparer la valeur numérique calculée dans l'étape (ii) avec des valeurs numériques obtenues à partir d'échantillons prélevés chez un ou plusieurs sujet(s) en bonne santé ou chez une ou plusieurs patiente(s) atteinte(s) d'un cancer du sein, chacune de ces valeurs numériques étant calculée, en tant que niveau normalisé de micro-ARN (α), par division d'un niveau du micro-ARN (α) par un niveau du micro-ARN (β), pour tester si oui ou non il y a une différence statistiquement significative entre ces valeurs numériques,

et déterminer par là si oui ou non le sujet est atteint d'un cancer du sein.

3. Procédé conforme à la revendication 1 ou 2, dans lequel le micro-ARN (α) est un micro-ARN d'au moins un type choisi parmi les suivants : hsa-miR-92a1, hsa-miR-92a2, hsa-miR-22, hsa-miR-370, hsa-miR-601 et hsa-miR-658.

4. Procédé conforme à l'une des revendications 1 à 3, dans lequel le micro-ARN (β) est un micro-ARN d'au moins un type choisi parmi les suivants : hsa-miR-638, hsa-miR-630 et hsa-miR-572.

5. Procédé conforme à l'une des revendications 1 à 4, dans lequel la valeur numérique obtenue lors de l'étape (ii) est comparée avec des valeurs numériques obtenues à partir d'échantillons prélevés chez une ou plusieurs patiente(s) atteinte(s) d'un cancer du sein.

6. Procédé conforme à l'une des revendications 2 à 5, dans lequel la valeur numérique obtenue lors de l'étape (ii) est comparée avec des valeurs numériques obtenues à partir d'échantillons prélevés chez une ou plusieurs patiente(s) atteinte(s) d'un cancer du sein, et un cancer du sein est diagnostiqué chez le sujet si l'on établit, dans l'étape (iii), qu'il n'y a pas de différence statistiquement significative.

7. Procédé conforme à l'une des revendications 1 à 6, dans lequel l'étape de détection de l'expression d'un micro-ARN (α) et/ou d'un micro-ARN (β) est réalisée par un procédé d'analyse en micro-réseau, de transfert Northern blot et/ou de PCR en temps réel.

8. Procédé conforme à l'une des revendications 1 à 7, dans lequel l'ARN total est extrait par ultracentrifugation sur chlorure de césium et guanidine ou par procédé AGPC (acide-guanidinium-phénol-chloroforme).

9. Utilisation d'une trousse dans un procédé de test de cancer du sein conforme à l'une des revendications 1 à 8, pour laquelle la trousse comporte au moins un réactif pour dosage d'un micro-ARN qui inclut une amorce de PCR pour détection d'un micro-ARN (α) et une amorce de PCR pour détection d'un micro-ARN (β), et dans laquelle le micro-ARN (α) est choisi parmi les hsa-miR-92a1, hsa-miR-92a2, hsa-miR-22, hsa-miR-370, hsa-miR-601, hsa-miR-658 et hsa-miR-494.

10. Utilisation conforme à la revendication 9, dans laquelle le micro-ARN (β) est choisi parmi les hsa-miR-638, hsa-miR-630 et hsa-miR-572.

[FIG. 1]

miR-*92a/miR-638

[FIG. 2]

Ct VALUE

has-miR-*92a

- ■ HEALTHY SUBJECT SAMPLE A
- ● HEALTHY SUBJECT SAMPLE B
- ▲ HEALTHY SUBJECT SAMPLE C

DAYS

[FIG. 3]

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KRUPNICK JG ; BENOVIC JL.** The role of receptor kinases and arrestins in G protein-coupled receptor regulation. *Annu Rev Pharmacol Toxicol,* 1998, vol. 38, 289-319
- **AMBROS V.** MicroRNA pathways in flies and worms: growth, death, fat, stress, and timing. *Cell,* 2003, vol. 113, 673-6
- **GROSSHANS H ; SLACK FJ.** Micro-RNAs: small is plentiful. *J. Cell Biol.,* 2002, vol. 156, 17-21
- **NELSON P ; KIRIAKIDOU M ; SHARMA A ; MANIATAKI E ; MOURELATOS Z.** The microRNA world: small is mighty. *Trends Biochem. Sci.,* 2003, vol. 28, 534-40
- **LAGOS-QUINTANA M ; RAUHUT R ; LENDECKEL W ; TUSCHL T.** Identification of novel genes coding for small expressed RNAs. *Science,* 2001, vol. 294, 853-8
- **LAU NC ; LIM LP ; WEINSTEIN EG ; BARTEL DP.** An abundant class of tiny RNAs with probable regulatory roles in Caenorhabditis elegans. *Science,* 2001, vol. 294, 858-62
- **LEE RC ; AMBROS V.** An extensive class of small RNAs in Caenorhabditis elegans. *Science,* 2001, vol. 294, 862-4
- **MOURELATOS Z ; DOSTIE J ; PAUSHKIN S et al.** miRNP: miRNPs: a novel class of ribonucleoproteins containing numerous microRNAs. *Genes Dev.,* 2002, vol. 16, 720-8
- **LIM LP ; GLASNER ME ; YEKTA S ; BURGE CB ; BARTEL DP.** Vertebrate microRNA genes. *Science,* 2003, vol. 299, 1540
- *Genome Summary List of Genome Currently Available,* 27 March 2009, http://microrna.sanger.ac.uk/cgi-bin/targets/v5/genome.pl?order#by=genome#name
- **CALIN GA ; DUMITRU CD ; SHIMIZU M et al.** Frequent deletions and down-regulation of micro-RNA genes miR15 and miR16 at 13ql4 in chronic lymphocytic leukemia. *Proc Natl. Acad. Sci. U.S.A.,* 2002, vol. 99, 15524-9
- **MICHAEL MZ ; SM OC ; VAN HOLST PELLEKAAN NG ; YOUNG GP ; JAMES RJ.** Reduced accumulation of specific microRNAs in colorectal neoplasia. *Mol Cancer Res,* 2003, vol. 1, 882-91
- **LAWIE CHARLES et al.** *discloses the detection of elevated levels of tumor-associated micro-RNAs in serum of patients with diffuse large β-cell lymphoma,* 2008